# EUROPEAN PATENT APPLICATION

(11) **EP 1 671 644 A2**
(43) Date of publication of application: **21.06.2006**
(21) Application number: 05077902.4
(22) Date of filing: 21.07.1998
(51) Int. Cl.: A61K 38/46

(54) **Pharmaceutical compositions containing Lysostaphin alone or in combination with an antibiiotic for the treatment of Staphylococcal infections**

(30) Priority: 23.07.1997 US 53470 P
(62) Divisional of application: 98935954.2
(71) Applicant: AMBI Inc., Purchase, NY 10577 (US)
(72) Inventor: Goldstein, Beth P., West Islip New York 11795 (US); Climo, Michael W., Richmond, VA 23236 (US); Novick, Richard P., New York 10024 (US); Archer, Gordon L., Richmond, VA 23233 (US)
(74) Representative: Lucas, Brian Ronald

(57) **Abstract**

Lysostaphin is demonstrated to be a powerful ani-staphylococcal agent suitable for parental administration to mammals including humans. Low dosages, on the order of 0.5 - 45 mg/kg/day are sufficient to eradicate most staphylococcal infections. Lysostaphin is also effective against bacteria of this type which have developed resistance to conventional antibiotics such as penicillins and vancomycin. Lysostaphin analogues, such as variants and related enzymes, show similar activity.

## Description

### BACKGROUND OF THE INVENTION

### Field of the Invention

This invention pertains to the administration of lysostaphin for the purpose of treatment of staphylococcus infection in mammals, including humans, as well as pharmaceutical preparations used in said treatment. This invention also pertains to methods of addressing particular disease conditions, including staphylococcal endocarditis; staphylococcal bacteremia; and staphylococcal infection of kidneys, lungs, skin, bone, burns, wounds and prosthetic devices. The invention embraces the use of lysostaphin broadly, including not only wild type lysostaphin but recombinant lysostaphin; lysostaphin variants with amino acid sequences varying from the published 'natural sequence' of the mature peptide (U.S. Patent No. 4,931,390) due to genetic mutations (such as substitutions, additions and deletions), posttranslational processing, genetic engineering of chimeric fusion proteins and the like or a combination of these kinds of variations.

### Background of the Prior Art

Lysostaphin is an enzyme, first identified in *Staphylococcus simulans* (formerly known as *S. staphylolyticus*). which has antimicrobial activity by virtue of its proteolytic activity on glycine-containing bridges in the cell wall peptidoglycan of bacteria [Zygmunt, et al., Progr. Drug Res. 16:309-333 (1972)].

*In vitro,* lysoscaphin is particularly active against *Staphylococcus aureus,* because the cell wall bridges of this species contain a high proportion of glycine, although activity against other species of staphylococci has been demonstrated (Ibid.).

The activity of lysostaphin has also been examined in animal infection models. Studies in which intraperitoneal treatment followed intraperitoneal infection are similar to *in vitro* experiments and are not considered here. There have been two reports of survival of 50% of treated mice when the animals were subjected to intraperitoneal infection followed by single or multiple subcutaneous administrations with a total of approximately 1 mg/kg of a lysostaphin preparation [Schuhardt, et al., J. Bacteriol. 88:815-816 (1964); Harrison, et al., Can. J. Microbiol. 13:93-97 (1967)]. A total dosage of 6 mg/kg was reported to protect 100% of the mice in one of these studies [Harrison, et al., Ibid.]. The virulence of the bacterial challenge used in both studies appears to be quite low, as the untreated infected mice did not all die within a short period of time.

Several experiments used a mouse subacute model measuring the bacterial load in the kidneys after infection with the Giorgio strain of *S. aureus* [Dixon, et al., Yale J. Biol. Med. 41:62-68 (1968); Schaffner, et al., Yale J. Biol. Med. 39:230-244 (1967); Harrison, et al., J. Bacteriol. 93:520-524 (1967)]. When a lysostaphin preparation was administered intravenously within 6 hours after infection, significant reductions in the numbers of bacteria in the kidneys were observed with dosages of 1.5 mg/kg or higher. However, established infections were more refractory; only modest reductions in the numbers of bacteria were seen when treatment was withheld for 24 hours or longer, even with dosages of 125 or 250 mg/kg of a lysostaphin preparation. The effect of multiple treatments was not studied.

A single study, Goldberg, et al., Antimicrob. Ag. Chemother. 1967:45-53 (1967), employed a limited number of dogs in an unusual endocarditis model. The dog model has not been further developed. The Goldberg, et al. experiment was not comparative, and is therefore of limited utility in assessment of the administration of lysostaphin. However, high dosages of lysostaphin (at least 50 mg/kg/treatment) were only moderately effective, as judged by the health of the dogs and by the extent of reduction in the number of bacteria in the heart valves and kidneys.

Accordingly, the data obtained from prior art studies with animal models do not teach that use of lysostaphin would be an effective and practical approach to clearing established infections from various organs.

Limited human trials were conducted aimed at eradication of nasal carriage of *S. aureus* by topical application of lysostaphin to the nares [Martin, et al., J. Lab. Clin. Med. 70:1-8 (1967); Martin, et al., J. Lab. Clin. Med. 71:791-797 (1968); Quickel, et al., Appl. Microbiol. 22:446-450 (1971)]. Nasal carriage is not in itself a disease state. It does constitute a risk factor for infection of patients treated by colonized health care professionals or for self-infection in the case of a colonized patient.

The art reports treatment of one very ill human patient with a single dose of parenterally administered lysostaphin, followed by an antibiotic, gentamicin, three days later. The patient died, but did exhibit a reduction in bacteremia [Stark, et al., N. Engl. J. Med. 291:239-240 (1974)].

Immunogenic phenomena observed during the course of the animal and human studies, were noted as a great concern. Contamination of the lysostaphin preparations with extraneous substances may have been responsible for at least some of these phenomena.

No further development of the enzyme as a therapeutic agent occurred, given the lack of desired effectiveness in the studies discussed. This may have been further due to the difficulty in producing and purifying lysostaphin.

The staphylococcal gene for lysostaphin has now been sequenced and cloned [U.S. Patent No. 4,931,390]. Lyostaphin for use as a laboratory reagent has been produced by fermentation of a non-pathogenic recombinant strain of *Bacillus sphaericus*, from which it is readily purified.

It remains an object of those of skill in the art to develop a therapeutic agent which can be administered parenterally and which can be used in the treatment of staphylococcal infection generally, as well as infection of specific tissues, as in endocarditis.

### SUMMARY OF THE INVENTION

The administration of relatively low dosages of lysostaphin (under 50 mg/kg) via parenteral administration is a dramatically effective therapy for the treatment of staphylococcal infections, particularly infections that are resistant to treatment, and/or typically associated with significant morbidity and mortality. Further, lysostaphin is demonstrated to be effective against staphylococcal bacteria that are at least partially resistant to available antimicrobial agents, such as beta-lactam antibiotics including penicillinase-stable penicillins, vancomycin, etc.

The invention further includes combination therapies comprising alternating or simultaneous administration of lysostaphin and one or more other antimicrobial agents. Particularly preferred antibiotics for administration in concert with lysostaphin according to this invention are rifamycins (isolated from microorganisms or synthetically or semi-synthetically produced, such as rifampin) and glycopeptides (a group of molecules among which the naturally occurring molecules usually contain a heptapeptide and one or more sugar moieties), whether naturally produced and isolated (such as vancomycin, teicoplanin, etc.) or semisynthetic preparations.

The availability of cloned, recombinant and variant lysostaphins further expands this invention. Related enzymes have been identified, and can further be used together with, or in place of, lysostaphin.

The cloning and sequencing of the lysostaphin gene permits the isolation of variant enzymes that can have properties similar to or different from those of wild type lysostaphin. One such altered enzyme, bearing a single amino acid change and which was the result of our work, has been characterized and shown to have potent anti-staphylococcal activity *in vitro* and in an animal infection model.

Other lysostaphin analogues, including naturally occurring enzymes with sequence homology to lypostaphin and with endopeptidase activity, or even chimeric enzymes obtained by fusing the binding domain of one enzyme to the catalytic domain of another, will be potent agents capable of addressing difficult-to-treat bacterial diseases caused by staphylococci or other pathogenic bacteria.

### BRIEF DESCRIPTION OF THE DRAWINGS

Figure 1 is a graphical representation of the bactericidal activity of lysostaphin against a methicillin-resistant *S*. *aureus* strain, as compared with vancomycin.
Figure 2 is a graph reflecting the bactericidal activity of lysostaphin against a variety of *S. aureus* strains of differing antimicrobial resistance.

### Definitions

Terms used in this application are used, where possible, in the sense of their normal and typical usage. Certain terms are used to describe a class of actions or compounds, to provide a generic description of items or scientific phenomena that are logically grouped together.

**Lysostaphin analogue** - Any enzyme, including lysostaphin (wild type), any lysostaphin mutant or variant, any recombinant, or related enzyme that retains the proteolytic ability, *in vitro* and *in vivo*, of proteolytic attack against glycine-containing bridges in the cell wall peptidoglycan of staphylococci. Variants may be generated by post-translational processing of the protein (either by enzymes present in a producer strain or by means of enzymes or reagents introduced at any stage of the process) or by mutation of the structural gene. Mutations may include site-deletion, insertion, domain removal and replacement mutations. The lysostaphin analogues contemplated in the instant invention may be recombinantly expressed or otherwise.

**Parenteral** - Administration by injection, including intravenous, intramuscular, subcutaneous, intraorbital, intraspinal, intraperitoneal and by direct perfusion or delivery to organs or tissues through injection (e.g., intramedullary).

### DETAILED DESCRIPTION OF THE INVENTION

*Staphylococcus aureus* is a highly virulent human pathogen. It is the cause of a variety of human diseases, ranging from localized skin infections to life-threatening bacteremia and infections of vital organs. If not rapidly controlled, an *S*. *aureus* infection can spread rapidly from the initial site of infection to other organs. Although the foci of infection may not be obvious, organs particularly susceptible to infection include the heart valves, kidneys, lungs, bones, meninges and the skin in burn patients. Surgical or traumatic wounds, and any region in which a foreign body is present are also frequently infected. These infections, which may arise in the community or during a hospital stay, are a cause of significant morbidity and mortality, which may be as high as 60% in severe infections in certain populations, even when the best available treatment is used. Other species of staphylococci (coagulase-negative staphylococci such as *S. epidermidis)* are less virulent, but can colonize catheters or prosthetic devices; this can have devastating consequences, for example when the device is an implanted heart valve.

Resistance to available antimicrobial agents appears to emerge particularly easily in staphylococci, starting with penicillin resistance in *S. aureus*; this resistance emerged soon after the dawn of the antibiotic era. Virtually all staphylococcal infections, whether arising in the community or the hospital, are no longer susceptible to first-generation penicillins due to the production of penicillinase; strains that are also resistant to penicillinase-stable penicillins (such as methicillin) are also now a significant problem, particularly in hospital-acquired infections. [Centers for Disease Control and Prevention, 1997. Reduced susceptibility of *Staphylococcus aureus* to vancomycin -- Japan, 1996. Morbidity and Mortality Weekly Report 46: 624-626(1997).]

Vancomycin has become the first-line treatment for staphylococcal infection, particularly in hospitals. However, as is evident from the high mortality rates, no currently available treatment is ideal for certain diseases, such as *S*. *aureus* endocarditis and bacteremia, which require rapid reduction in numbers of bacteria in order to prevent irreversible damage to the heart and to the other organs to which infection often spreads via the bloodstream. One reason for failure of currently available therapies is that they act relatively slowly, particularly *in vivo,* where rapid sterilization of infected sites may be required for complete and rapid recovery of the patient. In such a life-threatening situation, and in some other infections (for example in which treatment regimens are very lengthy, such as osteomyelitis), novel therapies or new combinations of therapies may greatly improve patient outcome.

Lysostaphin has been found to be highly active, at moderate doses. This is demonstrated, below, in a very severe well-characterized animal infection model, endocarditis in the rabbit caused by methicillin-resistant *S*. *aureus* (MRSA). In particular, we demonstrate complete sterilization of the heart valve vegetations in almost all animals treated with one of the dosage regimens, an unprecedented result not seen with currently available antimicrobial agents. We further demonstrate herein that combination of an even lower daily dosage of lysostaphin with a standard therapeutic agent potentiates the antimicrobial activity of the components in this model system.

The lysostaphin dosages we used were significantly lower than those previously demonstrated to have only a limited effect on clearance of bacteria from organs in animal models [Zygmunt et al, Progr. Drug. Res. 16:309-333 (1972); Goldberg et al, Antimicrob. Ag. Chemother. 1967:45-53 (1967)].

We have also demonstrated, below, activity against staphylococci, *in vitro* and in a mouse acute infection model, of an altered form of lysostaphin, generated by mutagenizing a recombinant strain of *Bacillus sphaericus* carrying the lysostaphin gene. It is therefore another realized aspect of the invention to administer pharmaceutical preparations of lysostaphin analogues, either lysostaphin or other enzymes with peptidoglycan endopeptidase activity, including genetically modified enzymes containing one or up to five amino acid substitutions; enzymes with deletions or insertions of up to 10 amino acids, including such deletions or insertions at the N-terminus; or chimeric enzymes that result from the fusion of the catalytic and binding domains of different enzymes, as therapeutic agents to treat infections in humans or animals.

For example, another glycylglycine endopeptidase (ALE-1, from *Staphylococcus capitis* EPK1) has been described. ALE-1 is distinct from lysostaphin, although the two enzymes have considerable amino acid homology [Sugai et al., J. Bacteriol. 179:1193-1202(1997)]. Another peptidoglycan hydrolase with a lower degree of homology to lysostaphin, but which also possesses endopeptidase activity, is zoocin A, produced by *Streptococcus zooepidemicus* 4881 [Simmonds et al., Applied and Environmental Microbiology 62:4536- 4541 (1996); Simmonds et al., Gene 189: 255-261(1997)]. Chimeric proteins can be produced by the fusion of a domain of these or similar enzymes to a domain of a lysostaphin analogue.

While certain immunologic side effects observed in much earlier studies may give concern in some, but not other situations (such as emergency or short term situations) suitably pure preparations of lysostaphin analogues, obtained by the fermentation of harmless recombinant strains of bacteria, are expected to be less prone to induce immunogenic or other side effects.

Effective pharmaceutical formulations of these antimicrobial enzymes include aqueous solutions or dry preparations (e.g., lyophilized, crystalline or amorphous, with or without additional solutes for osmotic balance) for reconstitution with liquids, suitable for parenteral delivery of the active agent. Delivery is preferably via intravenous (i.v.), intramuscular (i.m.), subcutaneous (s.c.), or intraperitoneal (i.p.) routes or intrathecally or by inhalation or by direct instillation into an infected site so as to permit blood and tissue levels in excess of the minimal inhibitory concentration (MIC) of the active agent to be attained and thus to effect a reduction in bacterial titers in order to cure or to alleviate an infection.

Furthermore, the active lysostaphin analogue can be coadministered, simultaneously or alternating, with other antimicrobial agents so as to more effectively treat an infectious disease. Formulations may be in, or be reconstituted in, small volumes of liquid suitable for bolus i.v. or peripheral injection or by addition to a larger volume i.v. drip solution, or may be in, or reconstituted in, a larger volume to be administered by slow i.v. infusion. Agents to be coadministered with lysostaphin or other antibacterial enzymes may be formulated together with said enzyme as a fixed combination or may be used extemporaneously in whatever formulations are available and practical and by whatever routes of administration are known to provide adequate levels of these agents at the sites of infection.

Suitable dosages and regimens of lysostaphin may vary with the severity of the infection and the sensitivity of the infecting organism and, in the case of combination therapy, may depend on the particular antimicrobial agent(s) used in combination. Dosages may range from 0.5 to 200 mg/kg/day, preferably from 3 to 25-50 mg/kg/day, given as single or divided doses, preferably given by continuous infusion or divided into two to four dosages per day.

### EXAMPLES

All experiments were conducted using lysostaphin analogues produced by fermentation of recombinant *B. sphaericus* strains engineered to contain the lysostaphin gene described by Recsei (U.S. Patent No. 4,931,390) or a mutant thereof. Specifically, the lysostaphin analogues prepared by fermentation of *B*. *sphaericus* varied from the published sequence by having as many as 2 fewer or up to 2 additional amino acids at the N-terminus.

In particular, the data herein are largely derived from studies using preparations of recombinantly produced lysostaphin analogues wherein the majority component is one that lacks the two N-terminal amino acids of the published sequence. However, the findings are not limited to these preparations. Similar results may be obtained with any preparation having suitable purity and activity.

### Example 1

### In Vitro Activity of Lysostaphin

As shown in Table 1a, experiments demonstrated that the lysostaphin preparation was active and bactericidal *in vitro* against clinical isolates of *S. aureus*; the minimal inhibitory concentrations (MIC) and minimal bactericidal concentrations (MBC) were determined to be ≤1.0 µg/ml using standard broth microdilution methods [National Committee for Clinical Laboratory Standards, 1993. Approved Standard M7-A3. Methods for dilution antimicrobial susceptibility tests for bacteria that grow aerobically - Third edition. National Committee for Clinical Laboratory Standards, Villanova, PA; National Committee for Clinical Laboratory Standards, 1992. Tentative Guideline M26-T. Methods for determining bactericidal activity of antimicrobial agents. National Committee for Clinical Laboratory Standards, Villanova, PA].

Furthermore, lysostaphin was shown to be active against a number of isolates of *Staphylococcus epidermidis* (a coagulase-negative species) with MIC ≤8 µg/ml for 11 of 13 clinical isolates tested. The MIC was defined as the lowest concentration tested that completely inhibited visible growth of the bacteria and the MBC as the lowest concentration that killed 99.9% of the initial inoculum in 24 hours of exposure. As shown in Table 1a, susceptibility to lysostaphin is not affected by resistance or reduced sensitivity to methicillin and/or vancomycin. *S. aureus* strains that are methicillin-resistant, and also have only intermediate susceptibility to vancomycin, have emerged recently in the U.S. [Centers for Disease Control and Prevention, Morbidity and Mortality Weekly Report 1997. 46:813-815].

**Table 1a. Preliminary study of in vitro susceptibility of S. aureus to lysostaphin**

| Strain | MIC (µg/ml) | MBC (µg/ml) |
|---|---|---|
| 1573^{c,m} | 0.5 | 1 |
| 27619^{c,m} | 0.25 | 0.5 |
| COL^{c,m} | 0.13 | 0.25 |
| 450M^{c,m} | 0.25 | 0.5 |
| 402^{c} | 0.5 | 0.5 |
| 404^{c,m} | 0.5 | 0.5 |
| 414 | 0.25 | 0.25 |
| 412 | 0.25 | 0.25 |
| 27286^{c,m} | 0.25 | 0.5 |
| 27698^{c,m} | 0.25 | 0.5 |
| 27222^{c,m} | 0.25 | 0.25 |
| 27287^{c,m} | 0.25 | 0.25 |
| 27295^{c,m} | 0.13 | 0.25 |
| 29293^{c,m} | 0.06 | 0.25 |
| 27621^{c,m} | 0.06 | 0.25 |
| 27622^{c,m} | 0.06 | 0.25 |
| 27619VR* | 0.5 | 0.5 |
| HP5827^{c,m,v} | 0.5 | 0.5 |
| HP5836^{c,m,v} | 0.5 | 1 |

| | | |
|---|---|---|
| ^{c}Clinical isolate; ^{m}ethicillin-resistant; ^{v}VISA strain (intermediate susceptibility to vancomycin); *strain with intermediate susceptibility to vancomycin derived, in the laboratory, from a methicillin-resistant clinical isolate. | | |

Lysoscaphin sticks to plastic materials and can be lost from solution; this can affect its apparent activity. Therefore, some MIC determinations were also performed with additions of 0.1% bovine serum album (BSA) to the diluent. Otherwise, the method was identical to that cited above. As shown in table 1b, the *in vitro* activity of lysostaphin against the strains tested improved by 8- to 64-fold when tested in the presence of BSA. Since this observation is related to the affinity of lysostaphin for plastic materials, it is to be expected that, in general, staphylococcal strains are more susceptible to lysostaphin than was observed previously.

**Table 1b. Activity of lysostaphin against S. aureus with and without BSA**

| | MIC (µg/ml) | |
|---|---|---|
| Strain | With BSA | Without BSA |
| 417 | 0.03 | |
| 414 | 0.03 | 0.25 |
| 404^{c,m} | 0.008 | 0.5 |
| 401^{c} | 0.015 | 0.5 |
| 27619^{c,m} | 0.008 | 0.25 |
| 27295^{c,m} | ≤0.004 | 0.13 |
| 27287^{c,m} | 0.03 | 0.25 |
| 25756^{c,m} | 0.008 | |

| | | |
|---|---|---|
| ^{c}Clinical isolate; ^{m}methicillin-resistant | | |

These data demonstrate the very potent activity of lysostaphin against contemporary clinical isolates of multiply antibiotic-resistant *Staphylococcus aureus.*

The bactericidal activity of lysostaphin against *S. aureus* was also studied by means of time-kill experiments. In one experiment of this type, *S. aureus* strain AG461, a methicillin-resistant clinical isolate from Genoa, Italy, was inoculated into Mueller-Hinton broth (Difco) and grown at 37°C with gentle shaking until it reached approximately 10⁸ viable cells per ml (CFU/ml), as estimated from the absorbance of the culture at 600 nm. The culture was then diluted with fresh broth to approximately 10⁶ CFU/ml and 5 ml aliquots were placed in several different flasks for exposure to different concentrations of antibacterial agents. Incubation was continued, with gentle shaking, at 37°C, and samples were withdrawn at intervals for determination of viable cells. Serial 10-fold dilutions of the samples were made in sterile saline (0.9% NaCl in distilled water) and duplicate 0.1 ml aliquots of appropriate dilutions were plated on Tryptic Soy agar plates (Remel) using the agar inclusion method. (In this method, the aliquot to be plated is added to 2.5 ml of top agar, which is mixed and poured onto a plate. Top agar consisted of molten Tryptic Soy agar (Difco) diluted 2-fold with Difco Tryptic Soy broth to give a final agar concentration of 0.75%, w/v.) The plates were incubated for 24-48 hours at 36°C and the colonies were counted manually. All dilutions of lysostaphin were made in the presence of 0.1-0.2% BSA, to prevent adsorption of lysostaphin to plastic materials. Vancomycin (Sigma Chemical Co.) was diluted in sterile distilled water.

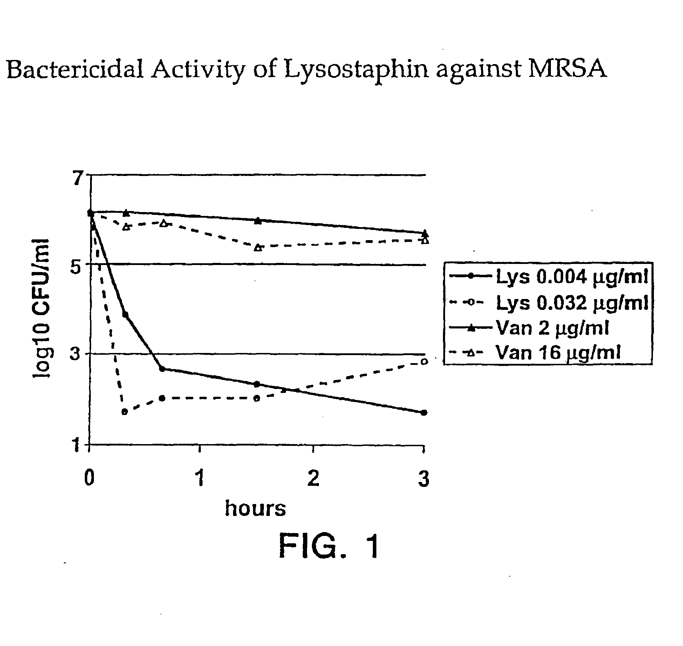

As shown in Figure 1, lysostaphin at concentrations of 0.004 and 0.032 µg/ml was rapidly bactericidal, with at least 99.9% of the bacteria being killed within one hour of contact.

In comparison, the bactericidal action of vancomycin was reduced and was much slower, with very little killing of the bacteria observed in three hours of contact, even though much higher concentrations of vancomycin (2 and 16 µg/ml) were used. The different concentrations of lysostaphin and vancomycin used were one and eight times their respective MIC.

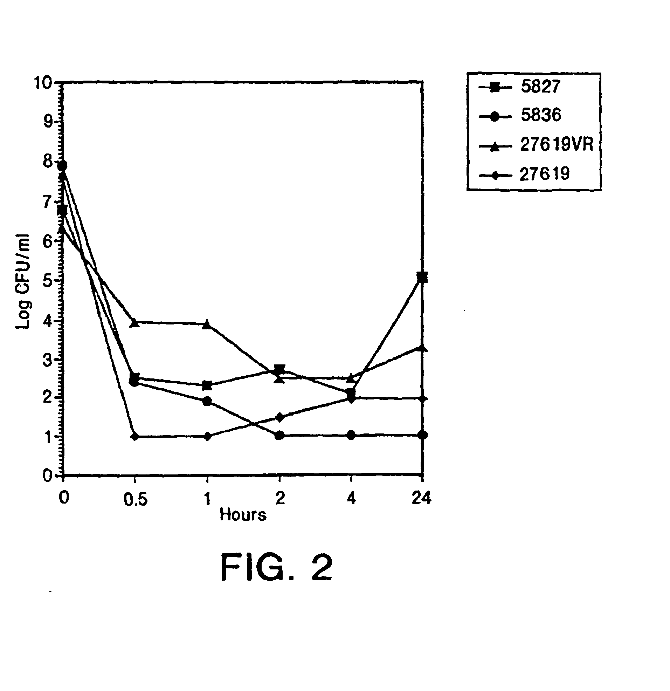

In another experiment (Figure 2), three different methicillin-resistant clinical isolates of *S. aureus,* and a fourth strain, 27619VR, a laboratory-derived 'VISA' strain (i.e., with intermediate resistance to vancomycin) derived from a methicillin-resistant clinical isolate, were inoculated into cation-adjusted Mueller-Hinton broth (Becton Dickinson) and grown at 37°C overnight. They were then diluted into fresh broth and incubated at 37°C with gentle shaking until they were estimated to have reached the logarithmic stage of growth. As indicated in Figure 2, the bacterial titers ranged from 2x10⁶ to 9x10⁷ CFU/ml at this time. Lysostaphin was added to each culture at the concentration of 1 µg/ml. At intervals, samples were withdrawn, serially diluted in 0.9% NaCl, and plated by spreading on Mueller-Hinton agar (Becton Dickinson). The agar plates were incubated for 48 hours at 37°C and the colonies were counted manually. As shown in Figure 2, all of these strains were rapidly killed by lysostaphin.

These data demonstrate that lysostaphin has potent and rapid bactericidal activity against contemporary clinical isolates of *S*. *aureus,* including strains resistant to methicillin and strains both resistant to methicillin and intermediately resistant to vancomycin.

### Animal Model Studies

### Example 2

### Comparative efficacy of lysostaphin in a mouse S. aureus infection model

The efficacy of lysostaphin was compared to that of vancomycin in an acute infection model in mice. *S. aureus* Smith was cultured overnight, with moderate agitation, in Veal Infusion broth (Difco) and diluted in broth containing 5% hog gastric mucin (Difco). Male Swiss~Webster mice (Taconic Farms, Germantown, NY) weighing approximately 20 grams were infected intraperitoneally with 10⁵-10⁶ viable cells, approximately tenfold the inoculum that reproducibly killed all untreated animals within 48 h. There were six mice in each treatment group. Lysostaphin was administered intravenously (in 0.1 ml 5% dextrose for injection) or subcutaneously (in 0.2 ml), within 10 min of infection. Vancomycin was administered subcutaneously.

As shown in Table 2, lysostaphin protected 100% of the infected mice when given at a dosage of 0.16 mg/kg intravenously or at a dosage of 2.5 mg/kg when administered subcutaneously. Vancomycin, which in the mouse is completely bioavailable subcutaneously, and has similar activity whether given subcutaneously or intravenously, was 100% effective at the dosage of 2.5 mg/kg. All of the untreated mice died in less than 24 hours.

**Table 2. Efficacy of lysostaphin against S. aureus infection in mice**

| Dose (mg/kg) | % survival | | |
|---|---|---|---|
| | lysostaphin iv | lysostaphin sc | vancomycin |
| 0 | 0 | 0 | 0 |
| 0.08 | 33 | | |
| 0.16 | 100 | | |
| 0.31 | 100 | | |
| 0.63 | 100 | | 0 |
| 1.25 | | 67 | 83 |
| 2.5 | | 100 | 100 |
| 5 | | 100 | 100 |
| 10 | | 100 | |
| 20 | | 100 | |

This example demonstrates that lysostaphin is effective against *S*. *aureus* infection in an acute infection model in mice using a highly virulent challenge dose of bacteria. When administered intravenously, exceedingly low doses of purified recombinant lysostaphin were effective. On a weight basis, lysostaphin was 16 times as effective as vancomycin; on a molar basis, lysostaphin was about 200 times as effective as vancomycin.

### Example 3

### In vitro and in vivo activity of a variant lysostaphin enzyme

A *Bacillus sphaericus* strain containing the cloned lysostaphin gene described in U.S. Patent No. 4,931,390 was mutagenized with N,N-nitrosoguanidine. Surviving colonies were screened for presence of a lytic activity by plating them on a lawn of heat-killed cells of *S. aureus* strain RN4880 and incubating overnight at 32°C. Colonies producing significant clear zones were saved.

One of these clones was further characterized. The lysostaphin gene was sequenced and found to contain a single G-to-A mutation in the codon corresponding to position 218 of the mature lysostaphin protein, resulting in a codon change from GGT (glycine) to GAT (aspartic acid). Fermentation of this mutant strain produced sufficient material for *in vitro* and *in vivo* testing.

As shown in table 3, the variant enzyme was highly active against *S. aureus in vitro*, although the wild type lysostaphin preparation was somewhat more active. In this experiment, MICs were determined by broth macrodilution in 1 ml final volumes in glass tubes. Otherwise, the methodology was as described above.

**Table 3. Activity of variant lysostaphin against S. aureus in vitro**

| | MIC (µg/ml) | | | |
|---|---|---|---|---|
| | AG417 | AG404^{c, m} | AG402^{c} | AG414 |
| Gly218Asp | .03 | .06 | .06 | .03 |
| wild type lysostaphin | .004 | .008 | .008 | .004 |

| | | | | |
|---|---|---|---|---|
| ^{c}clinical isolate; ^{m}methicillin-resistant | | | | |

As shown in table 4, the variant lysostaphin enzyme was also highly active against *S*. *aureus* in the acute mouse infection model. Here again, the variant was somewhat less active than the wild type lysostaphin, but it was more active than vancomycin.

**Table 4. Activity of variant lysostaphin against S. aureus infection in mice.**

| Dose (mg/kg) | % survival | | | |
|---|---|---|---|---|
| | Control | Lysostaph in | Gly218Asp | Vancomycin |
| 0 | 0 | | | |
| 0.04 | | 0 | | |
| 0.08 | | 17 | 0 | |
| 0.16 | | 83 | 17 | |
| 0.31 | | | 33 | |
| 0.63 | | | 83 | 0 |
| 1.25 | | | | 83 |
| 2.5 | | | | 100 |

### Example 4

### Antimicrobial activity in the serum of a rabbit treated with lysostaphin.

A New Zealand white rabbit weighing approximately 5 kg was given an intravenous infusion of 125 mg lysdstaphin. Blood samples were taken at intervals up to 4 h and serum was prepared; two-fold serial dilutions were made, and the serum bactericidal titer was determined against a methicillin-resistant strain of *S. aureus* (MRSA 27619). The serum bactericidal titer is the highest dilution that kills 99.9% of the inoculum in 24 h. In this test, survival of bacteria is determined essentially as in the minimal bactericidal method, except that the microtiter wells contain different dilutions of the serum, rather than different concentrations of a solution of a purified antimicrobial agent.

As shown in table 5, the serum contained highly bactericidal concentrations of lysostaphin over the entire period of time. In particular, at time points from 30 minutes to 120 minutes, the titer was greater than 1:256 (the highest dilution tested), indicating that dilutions of at least 256-fold were still able to kill 99.9% of the bacteria. At the latest time point, 240 minutes, the titer was 1:64.

**Table 5. Serum bactericidal titer of lysostaphin after administration of 125 mg to a 5-kg rabbit**

| Time after beginning of infusion (minutes) | Serum bactericidal titer |
|---|---|
| 0 | 1:128 |
| 30 | >1:256 |
| 60 | >1:256 |
| 90 | >1:256 |
| 120 | >1:256 |
| 240 | 1:64 |

This example demonstrates that lysostaphin maintains bactericidal activity in the serum of rabbits and that it remains present and active in the circulation for at least 4 hours after injection.

### Example 5

### Efficacy of lysostaphin against experimental endocarditis in rabbits.

Aortic valve endocarditis was established in New Zealand white rabbits weighing approximately 3 kg. Rabbits were anesthetized and the right carotid artery surgically exposed and cannulated with a polyethylene catheter which was advanced into the left ventricle of the heart. After at least 24 h, the rabbits were infected intravenously with 10⁶-10⁷ cells of a methicillin-resistant *S. aureus* strain (MRSA 27619). Twenty-four hours later, the animals were randomly assigned to different treatment groups: untreated control (9 rabbits); positive control, vancomycin 30 mg/kg twice daily (15); lysostaphin 5 mg/kg three times daily (11); lysostaphin 5 mg/kg once daily (10); lysostaphin 5 mg/kg once daily + vancomycin 30 mg/kg twice daily (11). Any rabbits whose infection was not confirmed by pre-treatment blood culture were eliminated. In addition, all rabbits included in the analysis were confirmed at autopsy to have had an established endocarditis infection, as judged by the presence of an aortic vegetation indicative of an ongoing or a previously existing disease state.

All treatments were intravenous and were carried out for three days. The state of health of the rabbits was assessed at intervals. The rabbits were sacrificed 18 h after the last treatment. Aortic vegetations were removed and weighed and processed to determine the number of viable bacteria, expressed as log₁₀CFU/gram. The limit of detection is 10² CFU/gram (log₁₀CFU/gram=2.0). The mean titers of bacteria per gram were compared by one-way analysis of variance. The Student-Newman-Keuls test was used to adjust for multiple comparisons. Comparison of the rates of sterilization were made using Fisher's exact test. Statistical significance was defined as a P value of ≤0.05.

As shown in table 6, a regimen of 5 mg/kg lysostaphin three times daily was the most efficacious treatment. An impressive statistic is that this treatment completely sterilized the heart valve vegetation in all but one of the rabbits. This was far superior to the standard regimen used as a positive control in this infection model: 30 mg/kg of vancomycin twice daily. A regimen of 5 mg/kg lysostaphin once daily was less efficacious than the thrice daily regimen, but was almost as good as vancomycin in reducing bacterial counts in the vegetation; in fact, the effect was not statistically different from the vancomycin group. The once-daily lysostaphin regimen also achieved complete sterilization of the vegetations in some animals. The addition of lysostaphin once daily to the standard vancomycin regimen produced a dramatic lowering in mean bacterial count, almost to the level seen with 3 daily lysostaphin treatments. However, in terms of the number of vegetations completely sterilized, the three-times-daily lysostaphin regimen was clearly superior to all others.

**Table 6. Efficacy of lysostaphin against S. aureus endocarditis in rabbits**

| Treatment | | Mean log₁₀CFU/gram of vegetation ± standard deviation | Number of sterile vegetations /total animals treated |
|---|---|---|---|
| Untreated control | | 10.73 ± 1.58 | 0/9 |
| Vancomycin mg/kg | 30 | 5.91 ± 1.67^{a} | 0/15 |
| twice daily Lysostaphin mg/kg | 5 | 7.08 ± 3.74^{a} | 2/10 |
| once daily Lysostaphin mg/kg | 5 | 0.85^{a,b} 2.26 ± 0.85^{a,b} | 10/11^{c} |
| three times daily Lysostaphin mg/kg once daily vancomycin mg/kg twice daily | 5 + 30 | 3.23 ± 1.41^{a,b} | 3/11 |

| | | | |
|---|---|---|---|
| ^{a}p<0.05 compared to untreated control; ^{b}p<0.05 compared to vancomycin; | | | |
| ^{c}p=0.008 vs lysostaphin once daily + vancomycin | | | |

Kidney abscesses were also assessed for the presence of staphylococci. The thrice-daily regimen of lysostaphin dramatically reduced the bacterial load as compared with the untreated control group to just over 10² CFU/gram of tissue in the lysostaphin group as compared with just under 10⁸ CFU/gram in the controls.

Observation of the animals demonstrated that rabbits treated with the thrice-daily regimen of lysostaphin were all in good health early in the treatment cycle.

These results could not have been anticipated on the basis of previous studies. In particular, sterilization of virtually all of the vegetations has never been seen or reported before with any antimicrobial agent in this infection model. The fact that sterilization occurred within a relatively short treatment period, 3 days, indicates that lysostaphin acts very rapidly *in vivo* and suggests that antimicrobial lysostaphin analogues could greatly improve the outcome in patients with serious staphylococcal infections that require rapid reduction in bacterial load.

The above data demonstrate the efficacy of lysostaphin analogues against *S. aureus,* including MRSA (methicillin-resistant *S. aureus*). Strains that are both methicillin-resistant and resistant to vancomycin are a newly emerging problem. A variant strain of this type was selected after cycles of growth in glycopeptide-containing medium. The vancomycin MIC for the resulting strain was 8 µg/ml, as reported also for naturally occurring VISA strains isolated from patients in the U.S. and Japan. (Centers for Disease Control and Prevention, Morbidity and Mortality Weekly Report 1997; 46:813-815). Staphylococcal isolates are considered to be susceptible to vancomycin if the MIC is less than or equal to 4 µg/ml and to be completely resistant if the MIC is greater than or equal to 32 µg/ml (National Committee for Clinical Laboratory Standards, 1993. Approved Standard M2-A5. Performance standards for antimicrobial disk susceptibility tests - Fifth edition. National Committee for Clinical Laboratory Standards, Villanova, PA.)

As shown in table 7, lysostaphin was efficacious in treating rabbits with infective endocarditis caused by the methicillin-resistant VISA strain.

**Table 7. Efficacy of lysostaphin against endocarditis in rabbits caused by a methicillin-resistant VISA strain of S. aureus**

| Treatment | CFU/g vegetation* | sterile/total vegetations |
|---|---|---|
| Control | 10.3 | 0/10 |
| Vancomycin 30 mg/kg | 6.95 | 0/13 |
| twice daily Lysostaphin 5 mg/kg | 6.29 | 2/10 |
| three times daily Lysostaphin 15 mg/kg twice daily | 4.0** | 0/5 |

| | | |
|---|---|---|
| *expressed as log10 of the mean. | | |
| **significantly better than vancomycin or the lower dose of lysostaphin (p<0.05) | | |

Against the VISA strain, lysostaphin at 5 mg/kg three times daily was as effective as vancomycin in reducing the bacterial load in aortic vegetations. Lysostaphin at 15 mg/kg twice daily was more effective than the standard dosage regimen of vancomycin (statistically significant) and also was significantly more effective than lysostaphin at 5 mg/kg given three times daily. Furthermore, vancomycin, even at 30 mg/kg twice daily, could not achieve complete sterilization of heart valve vegetations in any of the test animals. On the other hand complete sterilization was achieved in some animals with the three times daily regimen of lysostaphin.

The rabbit endocarditis model is now very well standardized and is accepted as a rigorous test of the ability of antimicrobial agents to cure severe human infections. Previous work with lysostaphin in established infections showed limited reduction in kidney bacterial load in a mouse model and in heart valves and other organs in a dog endocarditis model, at doses ranging from 50 to 250 mg/kg/treatment. Despite the high dosages used in these previous studies, effectiveness of the magnitude required in the treatment of severe staphylococcal infections was not observed. The results obtained previously would not have led to the prediction of the rapid, total sterilization of virtually all heart valve vegetations, as has now been seen using very moderate doses of lysostaphin in the rabbit endocarditis model.

The results presented herein demonstrate not only the unexpected effectiveness of lysostaphin against *S. aureus* endocarditis, but show that such efficacy is far superior to that expected for standard treatments. Currently available treatments are often not effective in dealing with life-threatening infections that may lead to irreversible tissue damage and that therefore require rapid reduction in bacterial numbers to prevent such damage as well as metastatic spread of infection to other vital organs. The above results indicate that lysostaphin analogues, alone or in combination with other agents, have the potential for effectiveness in the treatment of such infections. Furthermore, based on these results and on the *in vitro* activity of lysoscaphin against staphylococci, it is to be expected that lysostaphin analogues, alone or in combination with other agents, will be useful against species of staphylococci other than *S*. *aureus.* Among the agents suitable for use together with lysostaphin are vancomycin and other glycopeptides, rifampin and other rifamycins, and other anti-infective agents that have activity against staphylococci.

Lysostaphin analogues may be used not only in the treatment of staphylococcal endocarditis but other potentially lethal staphylococcal diseases, such as bacteremia and infections of other vital organs, such as kidneys, lung, skin and bone. The instant methods are also applicable to the treatment of infections of burns, wounds and prosthetic devices. These same methods may be used, in particular, in treatment of diseases such as osteomyelitis, which result from an infection of a type or severity requiring prolonged treatment with currently used antimicrobial agents. The instant invention further extends to the use of lysostaphin analogues in treating such infections and diseases when they are caused by staphylococci that are resistant to routinely used antibiotics.

## Claims

1. Use of lysostaphin, or a variant thereof that retains the ability of lysostaphin of proteolytic attack against glycine-containing bridges in the cell wall peptidoglycan of staphylococci, in the preparation of a pharmaceutical for parenteral administration to humans to treat staphylococcal infections, the administration being in a dosage given as divided doses.

2. Use according to Claim 1, wherein the staphylococcal infections are at least partially resistant to penicillinase-stable penicillins.

3. Use according to Claim 1 or 2, wherein the staphylococcal infections are at least partially resistant to methicillin.

4. Use according to any of Claims 1 to 3, wherein the administration is in a dosage of from 0.5 to 200 mg/kg/day.

5. Use according to any of Claims 1 to 3, wherein the administration is in a dosage of from 0.5 to 50 mg/kg/day.

6. Use according to any of Claims 1 to 3, wherein the administration is in a dosage of from 0.5 to 25 mg/kg/day.

7. Use according to any of Claims 1 to 6, in which the doses are divided into two to four doses per day.

8. Use according to any of Claims 1 to 6, in which the doses are given by continuous infusion.

9. Use according to any of Claims 1 to 8, wherein the lysostaphin or variant thereof is to be administered together with at least one other antimicrobial agent.

10. Use according to Claim 9, wherein the other antimicrobial agent is a rifamycin or a glycopeptide.

11. Use according to any of Claims 1 to 10, wherein the infection is in a heart valve, blood, kidney, lung, bone or meninges or is associated with a catheter or prosthetic device.

12. Use according to Claim 11, wherein the infection is endocarditis, osteomyelitis or bacteremia.

13. Use according to Claim 12, wherein the infection is endocarditis and complete sterilization of heart valve vegetations is achieved.

14. Use according to any of Claims 1 to 13, wherein the parenteral administration is by intravenous injection.

15. Use according to any of Claims 1 to 14, wherein the lysostaphin or variant thereof is recombinantly produced.
